Europäisches Patentamt

European Patent Office

Office européen des brevets

(11) Publication number: **0 353 209**
**A1**

## EUROPEAN PATENT APPLICATION

(21) Application number: **89830347.4**

(22) Date of filing: **25.07.89**

(51) Int. Cl.⁵: **A 61 B 8/08**
**A 61 C 19/04**

(30) Priority: **25.07.88 IT 6770588**

(43) Date of publication of application:
**31.01.90 Bulletin 90/05**

(84) Designated Contracting States:
**AT BE CH DE ES FR GB GR IT LI LU NL SE**

(71) Applicant: **GIEMMECI S.r.l.**
**Contrada Papazzo (Villa Caruso)**
**I-88046 Lamezia Terme (Catanzaro) (IT)**

(72) Inventor: **Conterno, Giuseppe Mario**
**Via Mazzini, 2**
**45100 Rovigo (IT)**

**De Angeli, Franco**
**Via Tripolitania, 3**
**31100 Treviso (IT)**

(74) Representative: **Bosotti, Luciano et al**
**c/o Jacobacci-Casetta & Perani S.p.A. Via Alfieri, 17**
**I-10121 Torino (IT)**

(54) **A method and a device for dental examination with ultrasound.**

(57) A method and a device for dental examination with ultrasound consisting in the scanning of the region under examination (C,B,D), which is effected by means of an array (10) of ultrasound transducers, with the possibility of shaping the beam to achieve an angle of incidence almost normal to the surfaces under examination and the further possibility of dynamically varying the focusing of the beam. The preferred application is for intraoperative endodontic diagnosis, particularly for detecting the position reached by intradental probes, for diagnosing conditions which involve morphological alterations in the tooth socket and for the morphological and clinical diagnosis of maxillary bone conditions of an orthopaedic nature.

FIG. 1

EP 0 353 209 A1

**Description**

## A method and a device for dental examination with ultrasound

### Technical background of the invention

The present invention relates to dental examination techniques and has been developed with particular concern for its possible use in intraoperative endodontic diagnosis, particularly for detecting the positions reached by intradental probes during operations such as canal treatment and the like.

Modern endodontics require the removal of the entire contents of the pulp cavities of teeth and their subsequent refilling with material such as cement, expanded gutta-percha, etc... as far as the root apex with a fairly close approximation. The variety of teeth as regards their lengths, diameters and root shapes (curved, stenotic, or at any rate irregular canals) prevents the standardisation of the operative technique and, on the contrary, necessitates the acquisition of data which can define a specific image, even with the aid of contrasting means introduced into the canal.

In current dental practice, this result is achieved by means of X-ray radioscopy. It is, however, well known that the X-rays used in dentistry are ionising and therefore harmful, exactly like those used in general medicine and in surgery.

In an attempt to remedy this problem, it has already been proposed to use ultrasound for dental examination, for example for the diagnosis of forms of osteoporosis by the detection of the pathological lowering of the bone density compared with normal values.

The use of ultrasound for dental examination, substantially like its use in other fields of medicine (for example abdominal echography, etc.), does, however, encounter some basic difficulties which result essentially from the heterogeneous nature of the medium through which the ultrasound beam must pass, with the consequent presence of a fairly high number of interfaces between regions of different compositions. The ultrasound beam can be reflected and refracted considerably at these interfaces and this can prevent its propagation into the regions to be investigated.

### Objects and summary of the invention

The object of the present invention is, therefore, to provide means for effecting dental examination, preferably in real time, particularly (but not exclusively) for the real-time display of the root or roots of an individual tooth and of its internal canals during operations for the treatment of the canals. In particular, the object of the present invention is to provide ultrasound means which enable the extent of insertion of probes into a dental canal, or the presence of morphological alterations in a tooth socket as a result of disease, to be displayed, thus replacing conventional radiographic means.

According to the present invention, these objects are achieved by virtue of a method and a device having the characteristics claimed in detail in the following claims.

In brief, the device according to the invention comprises means which can transmit and receive, by means of a suitable transducer (preferably produced in the form of a flat or linear matrix of transducer elements), ultrasound-frequency signals which are directed towards the tooth and diffused by a predetermined internal layer of material constituting the tooth or by any probe inserted therein.

### Detailed description of the invention

The invention will now be described, purely by way of non-limiting example, with reference to the appended drawings, in which:

Figures 1 and 2 show in side elevation and from above respectively, the position of use of a device according to the invention,

Figures 3 to 6 show in detail the structural configuration of one of the elements shown in Figures 1 and 2, and

Figure 7 finally shows, in the form of a block diagram, the general structure of a device according to the invention.

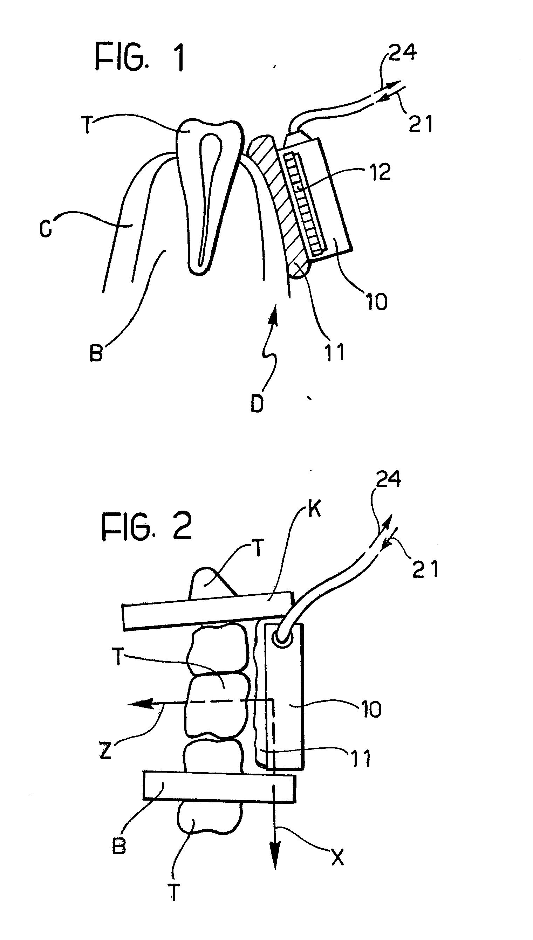

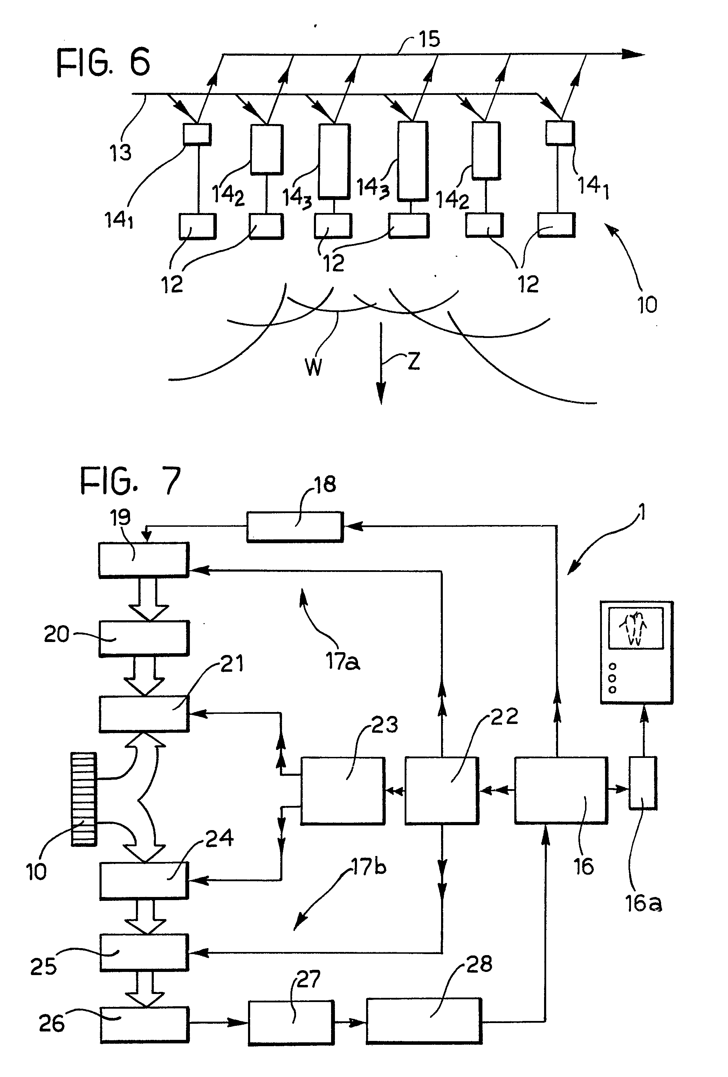

The device according to the invention, generally indicated 1 (Figure 7) includes, as an essential element, a transducer 10 which, in the embodiment illustrated, is constituted by a flat, two-dimensional array (matrix) 12 of ultrasound transducer elements. The transducer may be made by current techniques for producing ultrasound transducers used in other sectors of the art, such as, for example, the quality control of mechanical parts or the automatic guidance of underwater weapons. In practice, the transducer 10 is in the form of a square plate with dimensions of the order of 10 x 10 mm or less and a thickness of a few millimetres, which can be applied in contact with a dental arch D in accordance with the criteria illustrated schematically in Figures 1 and 2. Alternatively the transducer may be produced in the form of a linear array with associated means for moving it in the direction perpendicular to its length.

In these figures, reference numeral 11 indicates a coupling medium, constituted by a mass of material which is substantially transparent to ultrasound (such as, for example, silicone oils, water, wax, rubber) and which can fill the space between the flat front face of the transducer 10 and the outer surface of the dental arch without substantial loss of continuity. Within the dental arch can be distinguished a fleshy outer layer or gum C which covers the jawbone or mandibular body B in which the teeth T are fixed.

In use, the transducer 10 is kept in a position in which it is connected to the dental arch D by any known means such as fixing bridges K of the type currently used in the dental field. It can be important to keep the transducer 10 in a substantially fixed position relative to the tooth or teeth T under examination in order to enable differential checks to be carried out, for example for establishing, in real time, the position reached by an interdental probe within the dental canal by comparison with a previous image of the tooth taken before the insertion of the probe. Any displacement of the transducer 10 relative to the tooth between two successive checks could, in fact, lead to a display in which the position reached by the probe is erroneous, altered or at least confused.

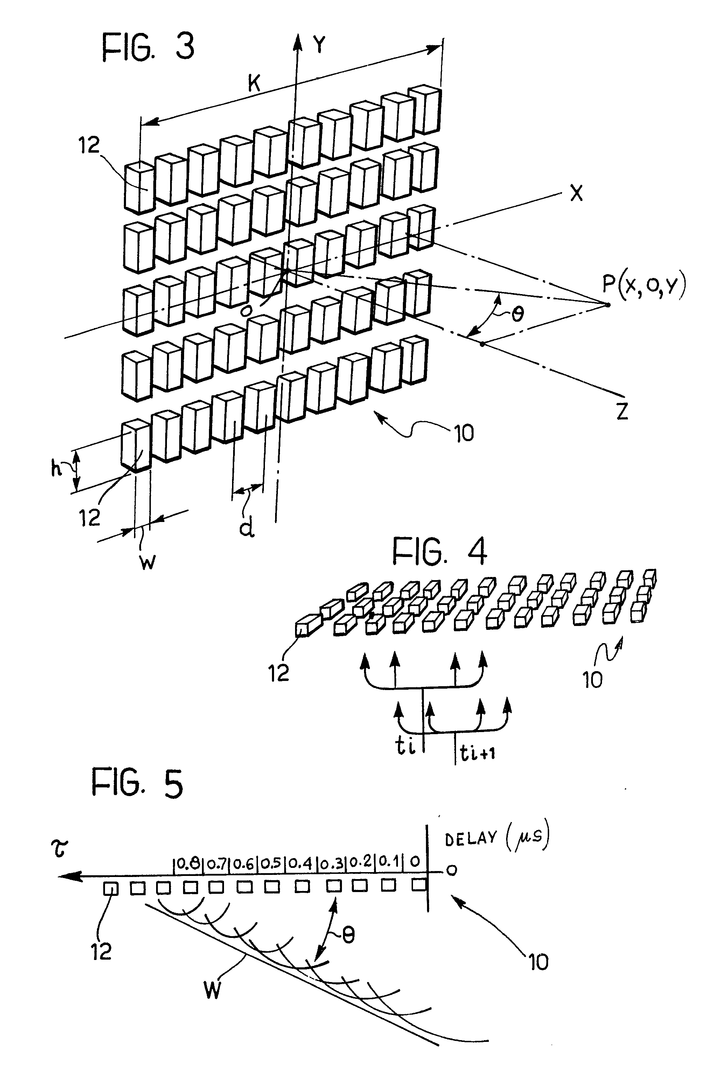

Figure 3 shows a possible embodiment of the transducer 10 in greater detail. In the example illustrated, it is assumed that the transducer in question is constituted by fifty transducer elements 12 arranged in five rows of ten elements of height h and width w, separated in each row by a distance d between the vertical median planes of each element.

In Figure 3 the matrix of transducer elements 12 has been shown with reference to a cartesian system (0, x, y, z) whose origin O is at the centre of the matrix of transducers 12 which are arranged in a plane (x, y) with the axis z perpendicular to the general plane of the matrix.

In general, the dimensions of the individual transducer elements 12 are selected so as to be sufficiently small for the focal region of the entire array to be located at a distance far from the element. In these conditions (according to well-known physical laws), if D is the desired focal distance and $\lambda$ indicates the wavelength (in the bony medium) then

$$h \sim W < 2\sqrt{\lambda D} \qquad (1)$$

With this hypothesis, it is possible to describe the ultrasound field generated by the transducer 10 by adding the contributions of the individual elementary transducers 12 according to the general formula

$$p = V_o \frac{J\rho ck \ h \cdot W}{2\pi R} e^{j\beta} e^{-(\alpha+jk)R} \sin c \frac{k(y-y')h}{2R} \cdot$$

$$\cdot \sin c \frac{k(x-x')w}{2R} \qquad (2)$$

where:
p = acoustic pressure
$V_o$ = surface velocity of the element
c = speed of sound in the medium
$K = 2\pi/\lambda$
$\rho$ = density of the medium
$\alpha$ = coefficient of attentuation of the medium
R = the distance of the element from the point being examined
$\beta$ = the phase of the surface velocity of the element.

By the suitable selection of the phase $\beta$ of each element, it is possible to achieve a maximum intensity of the ultrasound beam at the point in space which is desired at any time. In the currently-preferred embodiment of the invention, the individual elements 12 of the matrix transducer 10 are not all excited in every scanning cycle; only a subgroup is excited. This enables a more extensive region to be scanned in the direction identified by the axis x in Figures 2 and 3. For example, the scanning can be carried out in accordance with the scheme shown in Figure 4. In other words, in each cycle, only a subgroup of columns (4 out of 12 in Figure 4) is excited so that spatial translation of the beam is achieved. The use of scanning is necessary to scan more extensive regions than would be allowed solely by means of the deflection of the beam as described below.

Figure 5 shows schematically how the application of the excitation signal to the various transducer elements in each row or column with an increasing delay, enables a wave front W to be generated which is inclined at a particular angle $\theta$ to the general plane x, y of the transducer 10. In Figure 5 the increasing value of the delay applied to the various elements 12 is indicated schematically on the abscissa (from right to left). Thus Figure 6 shows, again in accordance with well-known criteria, how a spherical wave front W can be obtained by the application of the excitation (transmission) pulses to the separate elements 12 in a single row or column of the transducer matrix, the pulses being delayed in accordance with a general law of symmetry relative to the matrix. This result can be achieved, according to known criteria, by the interposition of delay lines $14_1, 14_2, 14_3$ having equally-spaced delay values $\tau_1, \tau_2, \tau_3$ between a main excitation line 13 and the individual elements 12, these delay lines carrying both the transmission pulses and the reception pulses sent by the transducer element 12 to a reception line 15. The delay lines $14_1, 14_2, 14_3$ may be produced by known technology, for example with the use of surface acoustic wave (SAW) devices or charge-coupled devices (CCD).

More generally, according to the basic principles of "synthetic aperture" (and/or phased array) techniques,

3

which are well known in the art, it is possible to obtain wave fronts W with geometries which are variable selectively within a wide range so as to compensate for the effects of the curvature of the various interfaces (gum C-bone B, bone B-tooth T, intradental interfaces, etc), so as to keep the direction of propagation of the ultrasound substantially perpendicular to the surface of the interface.

This factor is of considerable importance for the realisation of the invention. It may, indeed, be observed that the penetration of an ultrasound wave into a bony or dental material is greatly affected by the angle of incidence of the beam, until, at angles of incidence of a certain size, it is, in effect nil.

In the device of the invention, however, it is possible to keep the displacement of the angle of incidence of the ultrasound beam from the normal to the interface surface to considerably smaller values, certainly less than about 20°: for brevity, in the claims which follow, the criterion for the adjustment of the direction of propagation of the ultrasound beam has been expressed by the term "substantially perpendicular to the interface surface". Naturally, with the above premise, this term must not be interpreted narrowly: thus, in addition to displacements of fractions of a degree or of the order of several degrees from the normal to the interface surface, it also extends to the wider range defined above.

Another intrinsic characteristic of an array of elementary transducers 12 such as that used in the invention is to enable the beam to be focussed electronically at different focal distances for transmission and reception by means of the variation of the focal distance during reception by suitable processing means (both at the hardware and software levels) which process the excitation and reception signals applied to the individual elements 12.

Focussing is thus achieved which may be defined as dynamic: in other words, the focal distance during reception follows the progressive penetration of the wave front into the part under investigation. This enables the energy received at each depth to be maximised and a small, elongate focal region to be produced, which would be impossible with conventional focussing means.

The focussing can be effected along the two directions x and y of Figure 3.

The signals received by the individual elements 12 are amplified differently according to their location in the matrix of the transducer 10. The curve which represents their sensitivity as a function of the position of the element 12 in the matrix may follow any law of weighting (for example the laws of Gauss, Hamming, Parzen). At the moment a Hamming law is prefered and significantly reduces the incidence of lateral lobes while at the same time ensuring good focussing.

Figure 7 shows a block diagram of the device according to the invention.

The operation of the device 1 is controlled by a central processing unit 16 which pilots two chains, a transmission chain 17a and a reception chain 17b, which are connected to the transducer 10 as well as to a display unit 16a. The latter may be of any known type, preferably a colour graphics unit of the type currently used for CAD/CAM applications with the capacity to show the different compositions of the media scanned in the form of colour contrasts.

The transmission chain 17a comprises essentially a pulse transmitter 18 which is controlled by the unit 16 and which pilots the individual elements 12 of the matrix of the transducer 10 through a bank of transmission delay lines 19, an amplifier unit 20 and a multiplexer 21. The entirety is under the control of a deflecting/focussing logic unit 22 and a scanning logic unit 23 which use the general scanning criteria described above (Figures 3 to 6 and the relating description).

In a complementary manner, the reception chain 17b comprises a demultiplexer 24 which (under the control of the scanning logic unit 23 which also acts on the multiplexer 21) transfers the reception signals to a bank of reception delay lines 25. The signals, delayed in accordance with criteria set by the logic unit 22 (which also acts on the transmission delay lines 19) are fed to an adder or integrator 26 which can also perform the windowing or weighting function already described above. After amplification in an amplifier unit 27 and conversion of the wave form (A-scan) into digital form in a sampler 28 (with a sampling frequency approximately 10 times greater than the ultrasound frequency) the detection data are transmitted to the unit 16 for subsequent processing.

Naturally, and again according to know criteria, the transmission of signals from the delay lines 19 to the multiplexer 21 and from the demultiplexer 24 to the adder/integrator unit 26 is carried out in parallel on as many lines as there are transducer elements 12 which are simultaneously or almost simultaneously and independently supplied.

In general, the control unit 16 must coordinate the operation of the members subject to it so as to focus the beam at the desired depth in the region under examination.

The main parameters relating to the formation of the beam (depth of inspection, focal depth, size of the area scanned) can be set and modified by the operator so as to optimise the image obtained.

In its application to intraoperative endodontic diagnosis, the scanning and processing of the images is carried out by the unit 16 in accordance with following scheme:
- inspection with a long focal depth to detect the outline of the tooth by the detection, from each digitised scan, of the presence of the bone B- tooth T interface (see Figure 1) for each position of the beam and the consequent formation of an image of the tooth from which the outline of the tooth itself is extracted (by known enhancement algorithms);
- inspection with the focus adjusted to the distance (depth) of the canal in the absence of a probe inserted therein and with the storing of the matrix of scanning data relative to this condition;
- further inspection with the focus adjusted to the distance of the canal in the presence of a probe (or of any other means or instrument) inserted therein to a greater or lesser extent, with the formation of a further matrix of scanning data, and
- comparison by comparison of the differences between the matrices of scanning data and their subsequent display and presentation of the maximum grey level produced from the difference at each scan.

During real-time examinations, the last two stages are repeated cyclically so as to enable the operator to follow the evolution of the situation constantly.

The scanning of the beam enables the degree of definition of the image to be improved considerably, particularly when the so-called SAFT synthetic aperture techniques are used.

Particularly good experimental results have been achieved with the use of a transducer operating in the range 2 to 25 MHz and, in particular, with an operating frequency of 10 MHz and a band width of 5 MHz. A scanning pitch of 100 micrometers was selected.

## Claims

1. A method for carrying out dental examinations with ultrasound, characterised in that it comprises the steps of generating (10), in the region under examination, the region having at least one interface between zones of different composition (B, C, T) and a depth in the general direction of inspection (z), an ultrasound beam which satisfies at least one of the following characteristics:
- the direction of propagation of the beam is substantially perpendicular to the at least one interface, and
- the beam is subsequently focussed at values other than the said depth.

2. A method according to Claim 1, characterised in that the beam is generated by a phased array and/or synthetic aperture technique (10, 12) with the use of an array of ultrasound transducer elements (12).

3. A method according to Claim 2, characterised in that the array of transducer elements (12) is a flat array (10) which is kept substantially perpendicular to the general direction of inspection (z).

4. A method according to Claim 2, characterised in that the array of transducer elements (12) is a linear array which can be moved in a direction perpendicular to its length.

5. A method according to any one of Claims 1 to 4, characterised in that it includes at least two successive examinations.

6. A method according to any one of Claims 1 to 4, characterised in that it includes at least two successive examinations which are carried out while the geometry of propagation of the beam is kept substantially identical, with the subsequent differential evaluation of the results obtained in the two examinations which are carried out in the absence and in the presence respectively of an operative instrument or means in the region under examination.

7. A method according to Claim 1, characterised in that it includes the operation of focussing the ultrasound beam to different focal distances for transmission and reception, the focal distance being varied during reception so as to follow the progressive penetration of the ultrasound wave front into the region under examination.

8. A method according to Claim 5 or Claim 6, characterised in that it comprises the steps of:
- detecting the outline of a respective tooth (T) which is subject to examination by means of an ultrasound beam of long focal depth;
- carrying out a first inspection of a predetermined region of the tooth (T) with an ultrasound beam focussed at a predetermined depth which corresponds susbtantially to the position of the canal, with the subsequent storing of the data obtained in the first inspection, and
- carrying out a further inspection of the predetermined region with an ultrasound beam focussed at the predetermined depth in the presence of a dental instrument or means in the predetermined region, and comparing the data obtained during the further inspection with the data obtained in the first inspection.

9. A method according to Claim 8, applied to the carrying-out of dental examinations in real time, characterised in that it includes the repetition of the further inspection at subsequent times with successive comparisons of the data obtained in the further inspection with the data obtained in the first inspection.

10. A method according to Claim 8, characterised in that it includes the step of displaying the results of the comparisons of the inspection data obtained during the first and further inspections.

11. A device for carrying-out dental examinations with ultrasound, characterised in that it includes ultrasound generator and receiver means which can be applied in correspondence with a region under examination, the region having at least one interface between two zones of different compositions (B, C, D) and a depth in the general direction of inspection, the means being able to generate, in the region, an ultrasound beam which satisfies at least one of the following conditions:
- the direction of propagation of the beam is substantially perpendicular to the at least one interface, and
- the beam is subsequently focussed at different values of the depth.

12. A device according to Claim 11, characterised in that the means are constituted essentially by an array (10) of ultrasound transducer elements (12) which operate in accordance with a general "phased array" and/or synthetic aperture scheme (16 to 28).

13. A device according to Claim 12, characterised in that the array (10) of transducer elements (12) is a flat array which, in use, is kept substantially perpendicular to the general direction of inspection (z).

14. A device according to Claim 12, characterised in that the array of transducer elements (12) is a linear array with associated means for moving it in a direction perpendicular to its length.

15. A device according to Claim 11, characterised in that it includes fixing means (K) for keeping the ultrasound generator and receiver means (10) in a substantially fixed position relative to the region under examination.

16. A device according to Claim 11 or Claim 15, characterised in that it also includes means (11), which are substantially transparent to ultrasound, for coupling the ultrasound generator and receiver means (10) to the region under examination.

17. A device according to Claim 12, characterised in that it includes delay means (19, 25) for applying to the transducer elements (12) respective delays in the excitation signal being transmitted and/or in the transducer signal being received.

18. A device according to Claim 17, characterised in that it includes a unit (22), which can act on the delay means (19, 24), for controlling the deflection and focussing of the beam.

19. A device according to Claim 12 or Claim 17, characterised in that it includes multiplexing/demultiplexing means (21, 24) which act on the transducer elements (12) and a scanning control unit (23) which can control the operation of the multiplexing/demultiplexing means (21, 22) to effect a general matrix-scanning scheme.

20. A device according to Claim 12, characterised in that it includes weighting means (25) for applying a weighting law which is preferably selected from the group constituted by the Gauss, Hamming and Parzen weighting laws to the transducer signals provided by the transducer elements (12)

21. A device according to any one of Claims 11 to 20, characterised in that the ultrasound generator and receiver means operate in the frequency range 2 to 25 MHz, preferably around 10 MHz.

FIG. 1

FIG. 2

FIG. 3

FIG. 4

FIG. 5

FIG. 6

FIG. 7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | PATENT ABSTRACTS OF JAPAN, vol. 7, no. 48 (P-178)[1193], 24th February 1983, page 39 P 178; & JP-A-57 196 104 (MITSUBISHI DENKI K.K.) 02-12-1982 * Abstract * --- | 1,5,9, 11,15 | A 61 B 8/08 A 61 C 19/04 |
| A | | 16 | |
| Y | US-A-3 895 525 (C.W. EICHELBERGER et al.) * Abstract; column 4, line 16 - column 5, line 40; column 7, lines 37-68; column 9, line 34 - column 10, line 6; figures 1-9 * | 1,5,9, 11,15 | |
| A | | 2,3,7, 10,13, 17,18, 21 | |
| A | ULTRASONICS, vol. 9, no. 2, April 1971, pages 95-100; S: LEES et al.: "Looking into the tooth and its surfaces with ultrasonics" * Pages 95-97,99-100, sections: "Visualisation of subsurface tooth structure"; "Surface demineralisation"; figures 1,3,12 * --- | 1,5,6,9 -11,15, 16,21 | TECHNICAL FIELDS SEARCHED (Int. Cl.5) A 61 B A 61 C |

-/-

The present search report has been drawn up for all claims

| Place of search | Date of completion of the search | Examiner |
|---|---|---|
| THE HAGUE | 16-10-1989 | RIEB K.D. |

EPO FORM 1503 03.82 (P0401)

**European Patent Office**

**EUROPEAN SEARCH REPORT**

Application Number

EP 89 83 0347

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | I.E.E. PROCEEDINGS, vol. 134, no. 2, part A, February 1987, pages 179-187, Stevenage, Herts, GB; M. HALLIWELL: "Ultrasonic imaging in medical diagnosis" * Pages 180-182,186, sections: 2.1.2. "Electronic real time scanners", 2.2.2. "Electronic beam forming and dynamic focusing" and 4.1.1. "Arrays"; figures 3-5,13 * | 1-4,7, 11-14, 17-19, 21 | |
| A | US-A-4 305 296 (P.S. GREEN et al.) * Abstract; column 1, lines 33-66; column 2, line 30 - column 3, line 22; column 4, line 54 - column 5, line 47; figures 1-6 * | 1-2,4,7 ,11-15, 17-18 | |
| A | PATENT ABSTRACTS OF JAPAN, vol. 10, no. 117 (E-400)[2174], 2nd May 1986; & JP-A-60 253 197 (TOSHIBA K.K.) 13-12-1985 * Abstract * | 1,5,6,9 ,10 | |

**TECHNICAL FIELDS SEARCHED (Int. Cl.5)**

The present search report has been drawn up for all claims

| Place of search | Date of completion of the search | Examiner |
|---|---|---|
| THE HAGUE | 16-10-1989 | RIEB K.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)